# EUROPEAN PATENT SPECIFICATION

(11) **EP 3 449 838 B1**
(45) Date of publication and mention of the grant of the patent: **20.09.2023**
(21) Application number: 16899754.2
(22) Date of filing: 26.04.2016
(51) Int. Cl.: A61B 8/00, A61B 8/08

(54) **IMAGING METHOD AND DEVICE**
BILDGEBUNGSVERFAHREN UND VORRICHTUNG
PROCÉDÉ ET DISPOSITIF D'IMAGERIE

(43) Date of publication of application: 06.03.2019
(73) Proprietor: Telefield Medical Imaging Limited, Hong Kong (CN)
(72) Inventor: ZHENG, Yongping, Hong Kong (CN)
(74) Representative: Hellmich, Wolfgang
(86) International application number: PCT/CN2016/080261
(87) International publication number: WO 2017/185240

(56) References cited:
- EP-A1- 2 807 978
- CN-A- 1 759 812
- CN-A- 100 998 511
- CN-A- 102 125 443
- CN-A- 102 640 012
- CN-A- 102 688 066
- CN-A- 105 982 693
- JP-A- H06 245 937
- JP-A- 2014 121 434
- US-A- 5 787 889
- US-A1- 2005 228 250
- US-A1- 2008 267 479
- US-A1- 2011 021 914
- US-A1- 2011 079 082
- US-A1- 2015 182 191
- US-A1- 2015 351 725

## Description

The present application relates to the field of image processing, and more particularly, relates to an imaging method and device.

In the X-ray imaging mode, an X-ray beam is used to illuminate an area, and an image in the entire area is formed by projection. Similarly, magnetic resonance imaging (MRI) and computed tomography (CT) reconstruct the image of the entire area by collecting the data needed in a certain area.

In some imaging modalities, such as ultrasound imaging, optical coherence tomography, and terahertz imaging, imaging generally has one-dimensional to three-dimensional stages, such as single location with depth information, two-dimensional (2D) images, and three-dimensional (3D) images. A number of scan types, such as manual, mechanical or electronic scans, can be used to acquire a large number of low-dimensional images for constructing multi-dimensional images, for example, from 2D to 3D. In general, this is a relatively time consuming process, especially when the scan range is large (as opposed to a single frame imaging window). Therefore, these imaging methods are actually incremental. However, the incremental image features are invisible for most of the imaging time because the device shows the complete image after the sub-dimension image acquisition. At the same time, it takes a long time to use a probe for conducting 2D imaging to form a wide range of 3D ultrasound images.

US 2015/351725 A1 discloses a 3D ultrasound imaging system for assessing scoliosis. It teaches the patient's spine is scanned with the B-mode probe of the scanner to capture ultrasound images. The scan may cover any selected portion of the spine. The scanning length may be shortened depending on the area of curvature. Since a traditional X-ray assessment only provides a projection image, the system may receive greater acceptance from experienced examiners if it can also provide a projection image. Both X-ray assessment and the system may be used together where the system is frequently used for long term studies. Therefore, the system has the ability to view a projection image of marked features together with the ultrasound images. Furthermore, the system has the ability for X-ray images to be fused or combined together with the ultrasound measurement.

US 5787889A discloses a method of generating an ultrasound image of a three-dimensional target volume from a plurality of ultrasound data frames corresponding to a scan of the target volume. It teaches the acquisition task includes deriving position and orientation indicators for each gathered image frame. Volume reconstruction includes defining a reference coordinate system within which each image frame in a sequence of image frames is registered. The reference coordinate system is the coordinate system for a 3D volume encompassing the image planes to be used in the 3D image. The first image frame is used to define the reference coordinate system. As each image plane is registered, a 2D projection of the incremental volume is displayed. A shear-warp factorization process is used to derive a 2D projection for a rotated volume. A viewing transformation matrix is factorized into a 3D shear which is parallel to slices of the reference volume. A 2D warp then is implemented to produce the projection of the rotated volume. A target volume is incrementally reconstructed and displayed in real time on an ultrasound medical diagnostic imaging system. One advantage of incrementally constructing the target volume in real time is that the operator can see the target volume as it is being scanned. If the scan is too fast for example causing pockets of blank screen within the reference volume where data samples were not achieved, then the operator can go back over the empty areas by moving the transducer probe back over the corresponding area of the patient's anatomy. Thus, the operator can view the target volume and scan effectiveness in real time and improve the displayed images by deliberately scanning desired areas repeatedly or at a slower speed. By slower speed, it is meant that the operator moves the transducer probe over the patient more slowly.

US 2011/079082 discloses an ultrasonic diagnostic imaging system producing an extended field of view (EFOV) image. FIGS. 10a-10d of this document illustrate how a 3D EFOV image appears on the ultrasound system display as a patient is being scanned. As the probe is moved in the direction indicated by the arrow in FIG. 10a of US 2011/079082, image planes which bisect a blood vessel are successively added to the front surface of the volume being scanned and displayed. As the probe continues its movement along the skin of the patient, more and more planes are scanned and added to the front of the volume and the volume grows in this dimension. Each new plane is located with respect to the previous planes of the volume by estimating the motion and hence the displacement from the last acquired plane to the current one. This displacement information may be computed from the image data of one or more orthogonal scan planes. Each newly acquired plane is thus added in consideration of its correct geometrical relationship to the previously acquired planes and the volume. The resulting volume is thus geometrically accurate and capable of supporting quantified measurements of the structure shown in the volume.

US 2015/351725 A1 discloses an ultrasonic diagnosis apparatus and a medical image processing apparatus which are capable of displaying a three dimensional image by performing re-scanning for a region of interest. An operator, who may be a doctor, an inspector or a surgeon, scans a subject while sweeping the probe over the subject to thereby acquire a two-dimensional tomographic image. When a region of interest, which is considered to be a diseased part e.g., tumor site exists, the operator preferably clicks a mouse of the operation section to check the region of interest. After completion of scanning over a certain region, the operator uses position information of the probe acquired simultaneously with the scanning to construct a three-dimensional image from continuous two-dimensional tomographic images acquired by the sweeping of the ultrasonic probe. The subject is scanned with the probe swept over the subject, to thereby acquire the two-dimensional tomographic images. The three-dimensional image is constructed from the continuous two-dimensional tomographic images acquired by the sweeping. Subsequently the mark is set in a position to be scanned in more detail so as to select the segment region to be rescanned in detail. The rescanning is performed according to the mark information. In the rescanning, the marked region is scanned in more detail. After completion of the rescanning for the marked segment region, the three-dimensional image is automatically reconstructed. The operator determines whether or not the three-dimensional image acquired by the rescanning is satisfactory. When it is determined that the acquired three-dimensional image is not satisfactory, rescanning is performed once again. Alternatively, according to need, the mark may be reset. When it is determined that satisfactory images of the plurality of selected segment regions have been acquired, the scanning is ended. The image display processing section generates a two-dimensional ultrasonic image for display using the B-mode image data, Doppler image data, and the like output from the image data generation section. Further, the image display processing section generates a three-dimensional image from the two-dimensional ultrasonic image and displays the generated three-dimensional image on the display section.

JP 2014 121434A discloses how to guarantee the 3D ultrasound scanning route.

US 2015/182191A1 discloses a system and method for tracking completeness of coregistered medical image data.

EP 2807978A1 discloses an US-acquisition protocol with an interactive real-time feedback to the user.

CN 100 998 511 A discloses a real-time 3D ultrasonic imaging system with a free arm for clinic diagnosis.

The principle of target-based incremental imaging is not addressed in the prior art, and for any imaging, the most important is the target of imaging, such as tumor and spinal structures in ultrasound images.

The technical problem to be solved by the present application is to provide an imaging method and device for target-based incremental imaging in view of the deficiencies of the prior art.

This problem is solved by the subject matter of the independent claims.

Preferred embodiments of the invention are mirrored by the dependent claims.

The imaging device of the present application, further includes a device configured for when detecting that some part of the image of the target has not been imaged or the image quality of a part of the image of the target is poor, rescanning the part to make the part having good image quality.

In the imaging device of the present application, the data collecting device further includes a device configured for implementing a multi-angle two-dimensional scan of the target to obtain more image information about the target to enhance the image of the target.

In the imaging device of the present application, the target image forming device further includes a device configured for before arranging the plurality of image contents on an incremental sequence, further includes processing image smoothing and filtering between adjacent image contents.

In the imaging device of the present application, the target determining device includes a device configured for performing a two-dimensional or three-dimensional pre-scan within a certain range, and further includes an ultrasonic probe equipped with a three-dimensional locator.

The imaging method and device embodying the present application have the beneficial effects: preventing collection of unusable image information, shortening image data collection time, and increasing the speed of an imaging process. Meanwhile, in the present application, when scanning is performed, two-dimensional scanning is employed based on the target to form a three-dimensional image, which further reduces the time taken for imaging consumption. Further, in the scanning process of the present application, the image content of the target is displayed in real time to improve the quality of the image. When the quality of the image is poor, it is not necessary to wait until the scan and reconstruction are completed to rescan the entire target, further reducing the imaging time.
Figure 1 is a flow chart of an imaging method according to an embodiment of the present application;
Figure 2 is a diagram showing an example of a target in an imaging method according to an embodiment of the present application;
Figure 3 is an incremental spine image obtained by applying the imaging method of the present application to ultrasound imaging;
Figure 4 is a schematic diagram of an embodiment of an imaging device of the present application.

The present application provides an imaging method. In the imaging method, a target is first determined; then based on the target, during the scanning, by processing the image data and relevant spatial information of the target on a real-time basis, the image content of the target is obtained, and the image content of the target is displayed on a real-time basis, the obtained image content of the target is arranged on an incremental sequence to form the image of the target. The imaging method prevents collection of unusable image information, shortens image data collection time, and increases the speed of an imaging process. Meanwhile, in the present application, when scanning is performed, two-dimensional scanning is employed based on the target to form a three-dimensional image, which further reduces the time taken for imaging consumption. Further, in the scanning process of the present application, the image content of the target is displayed in real time to improve the quality of the image. When the quality of the image is poor, it is not necessary to wait until the scan and reconstruction are completed to rescan the entire target, further reducing the imaging time. Here, the image of the target is a surface image or a projected image.

In order to make the objects, technical solutions and advantages of the present application more comprehensible, the present application will be further described in detail below with reference to the accompanying drawings and embodiments. It is understood that the specific embodiments described herein are merely illustrative of the application and are not intended to limit the application.

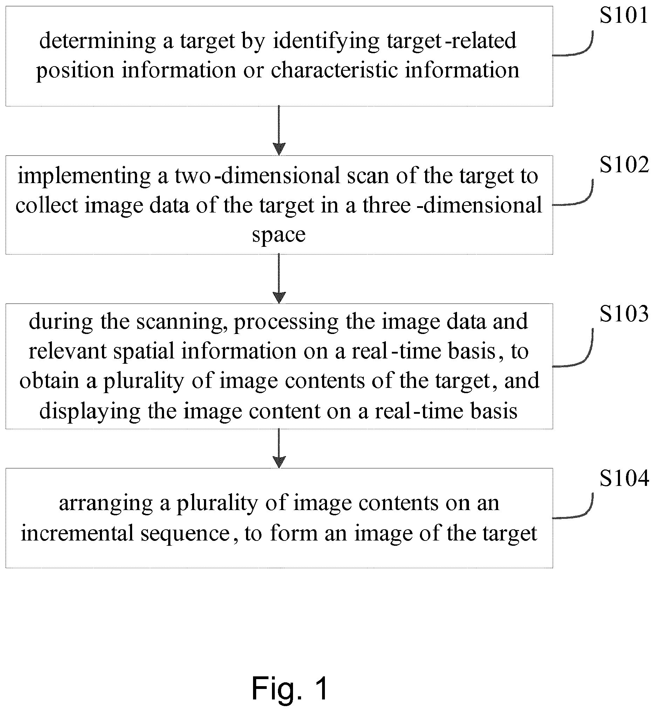

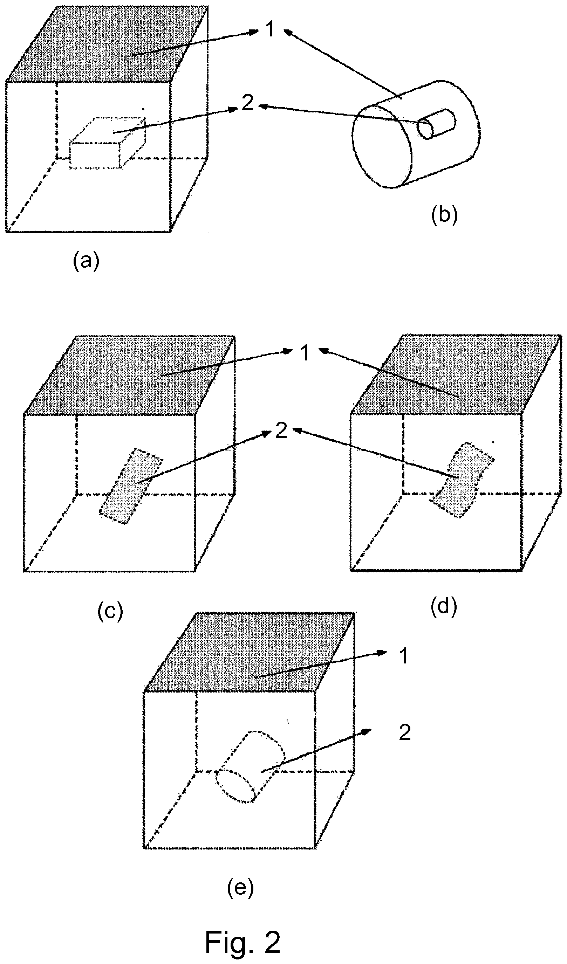

As shown in Figure 1, which is a flow chart of an imaging method according to an embodiment of the present application, the method includes the following steps:
S101, determining a target by identifying target-related position information or characteristic information;
In this step, the position information or the characteristic information are all predefined, wherein the position information is recorded by a known spatial positioning technique. The target is located in the three-dimensional space, for example, a tumor in the breast, a liver in the human body, a bone in the limb, a spine in the body, a plane or a curved surface inside the body. As shown in Figure 2, which is a diagram showing an example of a target in an imaging method according to an embodiment of the present application, 1 indicates the three-dimensional space in which the target is located, in Figure 2(a), the target 2 is a cuboid; in Figure 2(b), the target 2 is a curved surface body; in Figure 2(c), the target 2 is a plane; in Figure 2(d), target 2 is a curved surface; in Figure 2(e), target 2 is a cylinder. The target is also located in a two-dimensional space, such as a diseased tissue in an X-ray image

Here, the determination of the target is achieved by referring to the related image that has been obtained before.

S102, implementing a two-dimensional scan of the target to collect image data of the target in a three-dimensional space;
In this step, the scanning method can be manual scanning or mechanical scanning or mechanically assisted manual scanning. That is the two-dimensional scanning is realized by an ultrasonic probe equipped with a three-dimensional locator.

In another embodiment, the warning function may also be set in the above steps. That is when detecting that the position of the two-dimensional scan is not within a certain range of the target, automatically pausing and giving a visual or audible prompt and restarting after returning to the range of the target.

In another embodiment, an image enhancement link can also be set in the above steps. That is implementing a multi-angle two-dimensional scan of the target to obtain more image information about the target to enhance the image of the target.

S103, during the scanning, processing the image data and relevant spatial information on a real-time basis, to obtain a plurality of image contents of the target, and displaying the image content on a real-time basis;
In this step, the image content is a partial image of the target, and the real-time processing includes processing immediately after acquiring each image data, without waiting for all the images to be collected for processing. Wherein the image data comprises one frame or several frame images. Processing methods include pixel brightness post processing, image smoothing, and noise removal. Wherein, the pixel brightness post-processing includes a grayscale enhanced threshold window processing. In order to increase the contrast of the image, a gray threshold window is selected for enhancement, and the grayscale outside the gray threshold window is compressed or omitted. This allows the target to be highlighted on the image, improving the recognition of the target, usually using a linear transformation to enhance the grayscale. The image smoothing adopts the averaging method, i.e. lowpass filtering; the noise removal can be performed by the averaging method, including time averaging, spatial compounding and frequency fusion, etc. This part of the content belongs to the prior art and will not be described here. In this embodiment, the real-time processing further includes performing image reconstruction on the image content in the same or adjacent space immediately after obtaining one or several frames of images to get the image content representing the location and display it in real time according to the corresponding spatial location. Wherein, image reconstruction includes average processing.

S104, arranging a plurality of image contents on an incremental sequence, to form an image of the target.

In this step, before arranging the plurality of image contents on an incremental sequence, further includes implementing related processing between adjacent image contents for better images, and the related processing includes image smoothing and filtering operations, as well as image fusion of edges of adjacent image content.

In this embodiment, the order of incrementing is determined by the scanning order of the target.

In this embodiment, in the scanning process, after the determined image content is processed in real time, the image content in the preset time may also be displayed, and the image content in the plurality of preset times is arranged in an incremental manner according to the scanning order to form an image of the target. The reference point of the preset time may be some rhythmic physiological signals of the human body, such as the electrocardiogram signal of the human body. Of course, before the image content of the plurality of preset times is arranged in an incremental manner, the related image content may also be subjected to related processing. For this part, it has been described above, and details are not described herein again.

In another embodiment of the imaging method of the present application, when the target is selected, its approximate position and shape will be marked on the display device for reference in the two-dimensional scan described in S102.

In another embodiment of the imaging method of the present application, the imaging method further includes:
when detecting that some part of the image of the target has not been imaged or the image quality of a part of the image of the target is poor, rescanning the part and then proceeding to step S103 to make the part having good image quality. This interactive step, monitoring, can be monitored manually or by computer. In such a case, there is no need to rescan the entire target when scanning and reconstruction is completed, thereby reducing the time required for imaging.

In this embodiment, the imaging method further includes identifying multiple targets with different characteristic information for incremental imaging during scanning.

In this embodiment, the imaging method further includes:
if there is a plurality of different targets, during the scanning, transforming scanning the plurality of different targets.

For the imaging method provided by the present application, the imaging method will be described in detail below for the application of the imaging method to the B-mode ultrasound image of the back of the human body from the bottom end to the top end.

First, the target is determined, wherein the target is a plane defined by a curved surface relating to the morphology of the spine at a certain depth under the skin of the back surface.

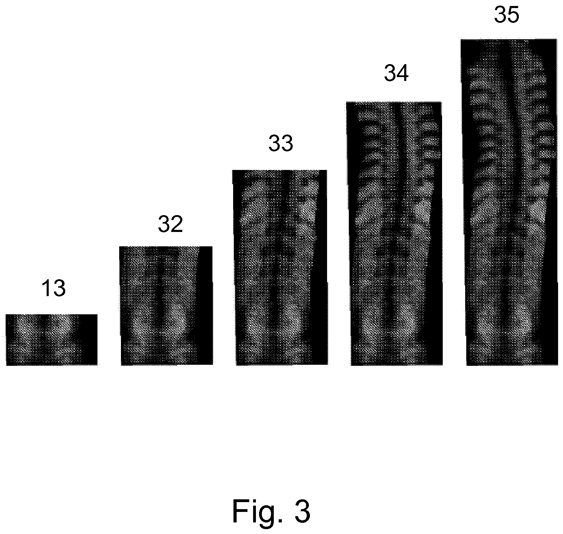

Secondly, the ultrasound probe in the ultrasound imaging system is used to scan the back of the human body. The main part of the ultrasound imaging system adopts the above imaging method to obtain an incremental spine image as shown in FIG. 3. As can be seen from the figure, viewed from left to right, the image content of the real-time display during the scanning process, respectively, has a first image 31, a second image 32, a third image 33, a fourth image 34, and a fifth image 35. And the latter image includes the previous image, that is, the image content is arranged in an ascending manner to form a complete spine image (i.e., the fifth image 35).

The imaging method of the present application can also be applied to other imaging modes with scanning.

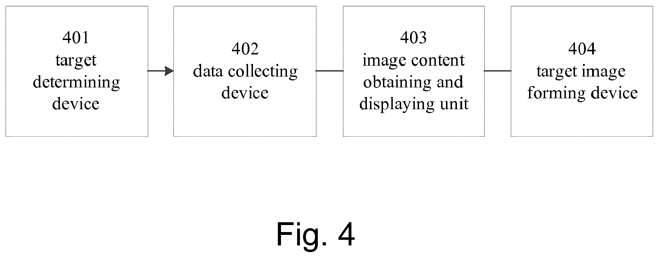

In the embodiment of the imaging device of the present application shown in Figure 4, the device includes a target determining device 401, configured for determining a target by identifying target-related position information or characteristic information; a data collecting device 402, configured for implementing a two-dimensional scan of the target to collect image data of the target in a three-dimensional space; an image content obtaining and displaying unit 403, configured for processing the image data and relevant spatial information on a real-time basis, to obtain a plurality of image contents of the target, and displaying the image content on a real-time basis during the scanning; and a target image forming device 404, configured for arranging a plurality of image contents on an incremental sequence, to form an image of the target.

The data collecting device 402 further includes a device configured for automatically pausing and giving a visual or audible prompt and restarting after returning to the range of the target when detecting that the position of the two-dimensional scan is not within a certain range of the target. The data collecting device 402 further includes a device configured for implementing a multi-angle two-dimensional scan of the target to obtain more image information about the target to enhance the image of the target.

The imaging device further includes a device configured for when detecting that some part of the image of the target has not been imaged or the image quality of a part of the image of the target is poor, rescanning the part to make the part having good image quality.

The target image forming device 404 further includes a device configured for before arranging the plurality of image contents on an incremental sequence, further includes processing image smoothing and filtering between adjacent image contents. The target determining device 401 includes a device configured for performing a two-dimensional or three-dimensional pre-scan within a certain range, and further includes an ultrasonic probe equipped with a three-dimensional locator.

In summary, in the imaging method and device of the present application, a target is first determined; then based on the target, during the scanning, by processing the image data and relevant spatial information of the target on a real-time basis, the image content of the target is obtained, and the image content of the target is displayed on a real-time basis, the obtained image content of the target is arranged on an incremental sequence to form the image of the target. The imaging method prevents collection of unusable image information, shortens image data collection time, and increases the speed of an imaging process.

## Claims

1. An imaging method, performed by an imaging device according to claims 14-15, wherein, the method comprises the following steps:
S1 (S101): determining a target by identifying target-related position information or characteristic information, the position information and the characteristic information are all predefined, the determination of the target is achieved by referring to a related image that has been obtained before;
S2 (S102): implementing a two-dimensional scan of the target to collect image data of the target in a three-dimensional space;
S3 (S103): during the scanning, processing the image data and relevant spatial information on a real-time basis, to obtain a plurality of image contents of the target, and displaying the image content on a real-time basis, the image content is a partial image of the target, and the real-time processing includes processing immediately after acquiring each image data, without waiting for all the images to be collected for processing;
S4 (S104): arranging a plurality of image contents on an incremental sequence, to form a three-dimensional image of the target.

2. The imaging method according to claim 1, wherein, in the step S2 (S102), the method further includes when detecting that the position of the two-dimensional scan is not within a certain range of the target, automatically pausing and giving a visual or audible prompt and restarting after returning to the range of the target.

3. The imaging method according to claim 2, wherein, the method further includes the following step: when detecting that some part of the image of the target has not been imaged or the image quality of a part of the image of the target is poor, rescanning the part to make the part having good image quality.

4. The imaging method according to claim 2 or 3, wherein, in the step S2 (S102), the method further includes implementing a multi-angle two-dimensional scan of the target to obtain more image information about the target to enhance the image of the target.

5. The imaging method according to one of the claims 2 - 4, wherein, in the step S4 (S104), before arranging the plurality of image contents on an incremental sequence, the method further includes processing image smoothing and filtering between adjacent image contents.

6. The imaging method according to one of the preceding claims, wherein, in the step S4 (S104), the order of incrementing is determined by the scanning order of the target.

7. The imaging method according to claim 6, wherein, the imaging method further includes: if there is a plurality of different targets, during the scanning, transforming scanning among the plurality of different targets.

8. The imaging method according to one of the preceding claims, wherein, in the step S3 (S103), the method further includes displaying the image content within a preset time.

9. The imaging method according to one of the preceding claims, wherein, in the step S1 (S101), the method further includes determining a plurality targets having different characteristic information.

10. The imaging method according to one of the preceding claims, wherein, in the step S1 (S101), the determination of the target is achieved by performing a two-dimensional or three-dimensional pre-scan within a certain range.

11. The imaging method according to one of the preceding claims, wherein, the two-dimensional scanning is realized by an ultrasonic probe equipped with a three-dimensional locator.

12. The imaging method according to one of the preceding claims, wherein, the image of the target is a surface image.

13. The imaging method according to one of the preceding claims, wherein, when the target is selected, its approximate position and shape will be marked on the display device for reference in the two-dimensional scan described in S2 (S102).

14. A imaging device, including:
a target determining device (401), configured for determining a target by identifying target-related position information or characteristic information, the position information and the characteristic information are all predefined, the determination of the target is achieved by referring to the related image that has been obtained before;
a data collecting device (402), configured for implementing a two-dimensional scan of the target to collect image data of the target in a three-dimensional space;
an image content obtaining and displaying unit (403), configured for processing the image data and relevant spatial information on a real-time basis, to obtain a plurality of image contents of the target, and displaying the image content on a real-time basis during the scanning, wherein the image content is a partial image of the target, and the real-time processing includes processing immediately after acquiring each image data, without waiting for all the images to be collected for processing; and
a target image forming device (404), configured for arranging a plurality of image contents on an incremental sequence, to form a three-dimensional image of the target.

15. The imaging device according to claim 14, wherein, the data collecting device (402) further includes a device configured for automatically pausing and giving a visual or audible prompt and restarting after returning to a certain range of the target when detecting that the position of the two-dimensional scan is not within the range of the target.

## Patentansprüche

1. Bildgebungsverfahren, das von einer Bildgebungsvorrichtung gemäß den Ansprüchen 14-15 durchgeführt wird, wobei das Verfahren die folgenden Schritte umfasst:
S1 (S101): Bestimmen eines Ziels durch Identifizieren von zielbezogenen Positionsinformationen oder charakteristischen Informationen, wobei die Positionsinformationen und die charakteristischen Informationen alle vordefiniert sind und die Bestimmung des Ziels durch Bezugnahme auf ein zugehöriges Bild erfolgt, das zuvor erhalten wurde;
S2 (S102): Implementieren einer zweidimensionalen Abtastung des Ziels, um Bilddaten des Ziels in einem dreidimensionalen Raum zu sammeln;
S3 (S103): während des Scannens Verarbeiten der Bilddaten und relevanter räumlicher Informationen auf Echtzeitbasis, um eine Vielzahl von Bildinhalten des Ziels zu erhalten und Anzeigen des Bildinhalts auf Echtzeitbasis, wobei der Bildinhalt ein Teilbild des Ziels ist und die Echtzeitverarbeitung eine Verarbeitung unmittelbar nach dem Erfassen aller Bilddaten umfasst, ohne darauf zu warten, dass alle Bilder zur Verarbeitung gesammelt werden;
S4 (S104): Anordnen mehrerer Bildinhalte in einer schrittweisen Folge, um ein dreidimensionales Bild des Ziels zu bilden.

2. Bildgebungsverfahren gemäß Anspruch 1, wobei das Verfahren in Schritt S2 (S102) ferner ein automatisches Anhalten und Ausgeben einer visuellen oder akustischen Aufforderung und Neustart nach der Rückkehr in einen bestimmten Bereich des Ziels erfolgt, wenn erkannt wird, dass die Position der zweidimensionalen Abtastung nicht innerhalb des Bereichs des Ziels liegt, umfasst.

3. Bildgebungsverfahren gemäß Anspruch 2, wobei das Verfahren ferner den folgenden Schritt umfasst: wenn erfasst wird, dass ein Bereich des Bilds des Ziels nicht abgebildet wurde oder die Bildqualität eines Bereichs des Bilds des Ziels schlecht ist, erneutes Scannen des Bereichs, um diesen Bereich in guter Bildqualität zu erhalten.

4. Bildgebungsverfahren gemäß Anspruch 2 oder 3, wobei das Verfahren in Schritt S2 (S102) ferner das Implementieren einer zweidimensionalen Mehrwinkelabtastung des Ziels umfasst, um mehr Bildinformationen über das Ziel zu erhalten, um das Bild des Ziels zu verbessern.

5. Bildgebungsverfahren gemäß einem der Ansprüche 2 bis 4, wobei das Verfahren in Schritt S4 (S104) vor dem Anordnen der Vielzahl von Bildinhalten in einer schrittweisen Folge ferner das Verarbeiten einer Bildglättung und Filterung zwischen benachbarten Bildinhalten umfasst.

6. Bildgebungsverfahren gemäß einem der vorangehenden Ansprüche, wobei in Schritt S4 (S104) die Reihenfolge der Schritte durch die Abtastreihenfolge des Ziels bestimmt wird.

7. Bildgebungsverfahren gemäß Anspruch 6, wobei das Bildgebungsverfahren ferner das Transformieren des Scannens unter der Vielzahl von unterschiedlichen Zielen während des Scannens umfasst, wenn eine Vielzahl von unterschiedlichen Zielen vorhanden ist.

8. Bildgebungsverfahren gemäß einem der vorangehenden Ansprüche, wobei das Verfahren in Schritt S3 (S103) ferner das Anzeigen des Bildinhalts innerhalb einer voreingestellten Zeit umfasst.

9. Bildgebungsverfahren gemäß einem der vorangehenden Ansprüche, wobei das Verfahren in Schritt S1 (S101) ferner das Bestimmen einer Vielzahl von Zielen mit unterschiedlichen charakteristischen Informationen umfasst.

10. Bildgebungsverfahren gemäß einem der vorangehenden Ansprüche, wobei im Schritt S1 (S101) die Bestimmung des Ziels durch Ausführen einer zweidimensionalen oder dreidimensionalen Vorabtastung innerhalb eines bestimmten Bereichs erreicht wird.

11. Bildgebungsverfahren gemäß einem der vorangehenden Ansprüche, wobei das zweidimensionale Scannen durch eine Ultraschallsonde realisiert wird, die mit einem dreidimensionalen Ortungsgerät ausgestattet ist.

12. Bildgebungsverfahren gemäß einem der vorangehenden Ansprüche, wobei das Bild des Ziels ein Oberflächenbild ist.

13. Bildgebungsverfahren gemäß einem der vorangehenden Ansprüche, wobei, wenn das Ziel ausgewählt ist, seine ungefähre Position und Form auf der Anzeigevorrichtung als Hinweis in der in S2 (S102) beschriebenen zweidimensionalen Abtastung markiert wird.

14. Bildgebungsvorrichtung, umfassend:
ein Zielbestimmungsgerät (401), konfiguriert zum Bestimmen eines Ziels durch Festlegen von zielbezogenen Positionsinformationen oder charakteristischen Informationen, wobei alle Positionsinformationen und alle charakteristischen Informationen vordefiniert sind, wobei die Bestimmung des Ziels durch Bezugnahme auf das zugehörige Bild erreicht wird, das zuvor erhalten wurde;
eine Datensammelvorrichtung (402), die zum Implementieren einer zweidimensionalen Abtastung des Ziels konfiguriert ist, um Bilddaten des Ziels in einem dreidimensionalen Raum zu sammeln;
eine Einheit (403) zum Erhalten und Anzeigen von Bildinhalten, die zum Verarbeiten der Bilddaten und relevanter räumlicher Informationen auf Echtzeitbasis konfiguriert ist, um eine Vielzahl von Bildinhalten des Ziels zu erhalten, und Anzeigen des Bildinhalts auf Echtzeitbasis während des Scannens, wobei der Bildinhalt ein Teilbild des Ziels ist, und die Echtzeitverarbeitung eine Verarbeitung unmittelbar nach dem Erfassen aller Bilddaten umfasst, ohne darauf zu warten, dass alle Bilder zur Verarbeitung gesammelt werden; und
eine Zielbild-Erzeugungsvorrichtung (404), konfiguriert zum Anordnen einer Vielzahl von Bildinhalten in einer schrittweisen Folge, um ein dreidimensionales Bild des Ziels zu erzeugen.

15. Bildgebungsvorrichtung gemäß Anspruch 14, wobei die Datensammelvorrichtung (402) ferner eine Vorrichtung umfasst, die zum automatischen Anhalten und Ausgeben einer visuellen oder hörbaren Aufforderung und Neustarten nach Rückkehr in einen bestimmten Bereich des Ziels konfiguriert ist, wenn erkannt wird, dass sich die Position des zweidimensionalen Scans nicht innerhalb des Bereichs des Ziels befindet.

## Revendications

1. Procédé d'imagerie, réalisé par un dispositif d'imagerie selon les revendications 14-15, dans lequel le procédé comprend les étapes suivantes :
S1 (S101) : détermination d'une cible en identifiant les informations de position associées à la cible ou les informations caractéristiques, les informations de position et les informations caractéristiques étant toutes prédéfinies, la détermination de la cible étant obtenue en référence à une image associée déjà obtenue au préalable ;
S2 (S102) : la mise en oeuvre d'un balayage en deux dimensions de la cible de façon à collecter des données d'image de la cible dans un espace tridimensionnel ;
S3 (S103) : pendant le balayage, le traitement des données d'image et des informations spatiales relatives sur une base en temps réel, pour obtenir une pluralité de contenus d'image de la cible et l'affichage du contenu d'image sur une base en temps réel, le contenu d'image étant une image partielle de la cible et le traitement en temps réel comprenant le traitement immédiatement après l'acquisition de chaque données d'image, sans attendre que toutes les images soient collectées pour les traiter ;
S4 (S104) : l'agencement d'une pluralité de contenus d'image sur une séquence incrémentielle, pour former une image tridimensionnelle de la cible.

2. Procédé d'imagerie selon la revendication 1, dans lequel à l'étape S2 (S102), le procédé comprend en outre qu'en cas de détection que la position du balayage en deux dimensions ne se trouve plus à une certaine portée de la cible, un signal sonore ou visuel se déclenche automatiquement et que le balayage reprend après retour dans la portée de la cible.

3. Procédé d'imagerie selon la revendication 2, dans lequel le procédé comprend en outre l'étape suivante : en cas de détection qu'une partie de l'image de la cible n'a pas été mise en image ou que la qualité d'image d'une partie de l'image de la cible est mauvaise, rebalayage de la partie de façon à obtenir une bonne qualité d'image pour la partie.

4. Procédé d'imagerie selon la revendication 2 ou 3, dans lequel à l'étape S2 (S102), le procédé comprend en outre la mise en oeuvre d'un balayage en deux dimensions à angles multiples de la cible pour obtenir davantage d'informations d'image sur la cible de façon à améliorer l'image de la cible.

5. Procédé d'imagerie selon l'une quelconque des revendications 2 à 4, dans lequel à l'étape S4 (S104), avant d'agencer la pluralité de contenus d'image sur une séquence incrémentielle, le procédé comprend en outre le traitement d'une atténuation et d'un filtrage de l'image entre des contenus d'image adjacents.

6. Procédé d'imagerie selon l'une quelconque des revendications précédentes, dans lequel à l'étape S4 (S104), l'ordre d'incrémentation est déterminé par l'ordre de balayage de la cible.

7. Procédé d'imagerie selon la revendication 6, dans lequel le procédé d'imagerie comprend en outre : en présence d'une pluralité de cibles différentes, pendant le balayage, transformation du balayage entre la pluralité de cibles différentes.

8. Procédé d'imagerie selon l'une quelconque des revendications précédentes, dans lequel à l'étape S3 (S103), le procédé comprend en outre l'affichage du contenu d'image pendant un temps prédéfini.

9. Procédé d'imagerie selon l'une quelconque des revendications précédentes, dans lequel à l'étape S1 (S101), le procédé comprend en outre la détermination d'une pluralité de cibles ayant des informations caractéristiques différentes.

10. Procédé d'imagerie selon l'une quelconque des revendications précédentes, dans lequel à l'étape S1 (S101), la détermination de la cible est obtenue en réalisant un pré-balayage bidimensionnel ou tridimensionnel à l'intérieur d'une certaine portée.

11. Procédé d'imagerie selon l'une quelconque des revendications précédentes, dans lequel la réalisation d'un balayage en deux dimensions est réalisé à l'aide d'un échantillon ultrasonique équipé d'un système de positionnement tridimensionnel.

12. Procédé d'imagerie selon l'une quelconque des revendications précédentes, dans lequel l'image de la cible est une image de surface.

13. Procédé d'imagerie selon l'une quelconque des revendications précédentes, dans lequel lorsque la cible est sélectionnée, sa forme et position approximative est marquée pour référence sur le dispositif d'affichage dans le balayage en deux dimensions décrit à l'étape S2 (S102).

14. Un dispositif d'imagerie, comprenant :
un dispositif de détermination de cible (401), configuré pour déterminer une cible en identifiant des informations de position associées à la cible ou des informations caractéristiques, les informations de position et les informations caractéristiques sont toutes prédéfinies, la détermination de la cible est réalisée en référence à l'image associée précédemment obtenue ;
un dispositif de collecte de données (402), configuré pour réaliser un balayage en deux dimensions de la cible de façon à collecter des données d'image de la cible dans un espace tridimensionnel ;
une unité de saisie et d'affic
hage de contenu d'image (403), configurée pour traiter les données d'image et les informations spatiales relatives sur une base en temps réel, pour obtenir une pluralité de contenus d'image de la cible, et l'affichage du contenu d'image sur une base en temps réel pendant le balayage, dans lequel le contenu d'image est une image partielle de la cible et le traitement en temps réel comprend le traitement immédiatement après l'acquisition de chacune des données d'image, sans attendre que toutes les images soient collectées pour les traiter ; et
un dispositif de formation d'image de cible (404), configuré pour agencer une pluralité de contenus d'image sur une séquence incrémentielle, de façon à former une image tridimensionnelle de la cible.

15. Dispositif d'imagerie selon la revendication 14, dans lequel le dispositif de collecte de données (402) comprend en outre un dispositif configuré pour s'arrêter automatiquement et transmettre un signal sonore ou visuel et redémarrer après être revenu vers une certaine portée de la cible grâce à une détection que la position du balayage en deux dimensions n'est pas à l'intérieur de la certaine portée de la cible.
